# EUROPÄISCHE PATENTSCHRIFT

(11) **EP 2 468 238 B1**
(45) Veröffentlichungstag und Bekanntmachung des Hinweises auf die Patenterteilung: **21.02.2018**
(21) Anmeldenummer: 11193492.3
(22) Anmeldetag: 14.12.2011
(51) Int. Cl.: A61K 8/04, A61K 8/81, A61K 8/893, A61Q 5/06

(54) **Volumengebender Haarfestiger unter Verbesserung der Sensorik**
Volumising hair fixer with improved sensors
Fixateur de cheveux volumisant avec amélioration de la technique sensorielle

(30) Priorität: 21.12.2010 DE 102010063786
(43) Veröffentlichungstag der Anmeldung: 27.06.2012
(73) Patentinhaber: Beiersdorf AG, 20253 Hamburg (DE)
(72) Erfinder: Franck, Kerstin, 25451 Quickborn (DE); Sors, Nathalie, 21543 Beckdorf (DE); Mahadeshwar, Anand, New Jersey, NJ New Jersey 07442 (US)

(56) Entgegenhaltungen:
- EP-A1- 1 269 974
- EP-A2- 1 048 286
- DE-A1- 4 421 562
- US-A1- 2003 147 828

## Beschreibung

Die Erfindung betrifft Volumengebende Haarfestiger mit verbesserter Sensorik.

Alle Mengenangaben sind- sofern nicht anders angegeben - Massenprozente bezogen auf die Gesamtmasse der Zubereitung.

Natürliches Kopfhaar ist entscheidend für das Erscheinungsbild und Selbstwertgefühl des Individuums. Haar hängt zumeist schlaff ohne Volumen herab. Zur temporären Verformung und Stabilisierung der Frisur werden Produkte eingesetzt, die als Stylingmittel bekannt sind. Hierzu zählen Haarsprays, Schaumfestiger, Gele, Wachse usw. Die Rezepturen sind vielfältig. Die Bestandteile müssen in ethanolischem, wässrig-ethanolischem oder wässrigem Medium verträglich sein.

Die wichtigsten Inhaltsstoffe dieser Haarfestiger stellen Filmbildnern und Lösungsmitteln dar. Üblicherweise bestehen diese Mittel zur Festigung der Haare aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation und nach Verdunstung des Lösungsmittels einen transparenten, farblosen Film, der die Fasern fixiert. Es werden eine Reihe von Anforderungen an diese Filme gestellt: Sie sollen klar, glänzend, feuchtigkeitsunempfindlich, festigend, langanhaltend, flexibel und nicht klebend sein, den natürlichen Griff des Haares nicht beeinträchtigen und sich auch wieder leicht durch handelsübliche Haarwaschmittel auswaschen lassen. Haarfestiger besitzen in der Gruppe der Stylingprodukte eine Sonderstellung. Nicht nur die Filmeigenschaften (Halt, Flexibilität) stehen im Vordergrund, sondern auch die Pflegeleistung am Haar (Kämmbarkeit, gepflegter Griff). Bei Schaumfestigern hat außerdem die Schaumcharakteristik (feinblasig, cremig, stabil) einen beträchtlichen Einfluss auf die Produktperformance. Üblicherweise enthalten Stylingmittel weitere kosmetische Hilfsstoffe mit unterschiedlichen Funktionen wie Pflegestoffe, Konservierungsmittel, Parfüme, Emulgatoren, Treibmittel, weichmachende, anfeuchtende und/oder feuchthaltende Substanzen, Fette, Öle, Wachse, Fettalkohole, Fettsäureester oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Als haltgebende Polymere in Haarfestigern finden hauptsächlich statistische Copolymere Verwendung. Copolymerisiert werden in der Regel verschiedene nichtionische, anionische oder kationische/kationogene Monomeren mit Vinyl- oder Acryl-Funktion, wie z. B. Vinyllactame (Vinylpyrrolidon, Vinylcaprolactam), Vinylacetat, Alkyl(meth)acrylate (Methyl(meth)acrylat, Ethyl(meth)acrylat, Propyl(meth)acrylat, n-Butyl(meth)acrylat, tert-Butyl(meth)acrylat, Cyclohexyl(meth)acrylat, usw.), Styrol, (Meth)Acrylamid, Alkyl(meth)acrylamide, funktionalisierte Alkyl(meth)acrylate (z. B. Hydroxyethyl(meth)acrylat, Dimethylaminoethyl(meth)acrylat), Dimethylaminopropyl(meth)acrylat), funktionalisierte Alkyl(meth)acrylamide (Dimethylaminopropyl(meth)acrylamid), (Meth)Acrylsäure, Maleinsäure, Crotonsäure, Acrylamidomethylpropansulfonsäure, aminogruppenhaltige Monomere, deren Aminogruppe alkyliert (quarternisiert) vorliegt, wie (Meth)Acryloylethyltrialkyl Ammoniumchlorid, (Meth)Acryloylpropyltrialkyl Ammoniumchlorid, (Meth)Acryloylaminopropyltrialkyl Ammoniumchlorid, (Meth)Acryloylethyltrialkyl Ammoniummethosulfat, (Meth)Acryloylpropyltrialkyl Ammoniummethosulfat, (Meth)Acryloylaminopropyltrialkyl Ammoniummethosulfat. Beispiele für in Haarfestigern verwendete Polymere dieser Art sind z.B. Polymere mit der INCI Acrylates Copolymer (Luvimer®, Luviflex® Soft von BASF, Balance® von National Starch, Avalure AC® von Lubrizol), Acrylates/Hydroxyesters Acrylates Copolymer (Acudyne® von Rohm & Haas), Acrylates/Octylacrylamide Copolymer (Amphomer® oder Resyn XP von National Starch), AMP-Acrylates/Allyl Methacrylates Copolymer (Fixate® von Lubrizol), Butyl Ester of PVM/MA Copolymer (Gantrez® von ISP), Ethyl Ester of PVM/MA Copoylmer (Gantrez®, Omnirez® oder Advantage® von ISP), Acrylamide/Sodium Acryloyldimethyltaurate/Acrylic Acid Polymer (Acudyne SCP® von Rohm & Haas), Isobutylene/Ethylmaleimide/Hydroxyethylmaleimide Copolymer (Aquaflex FX-64® von ISP), Octylacrylamide/Acrylates/Butylaminoethyl Methacrylate Copolymer (Amphomer® oder Balance® von Natinal Starch), Acrylates/t-Butylacrylamid Copolymer (Ultrahold® von BASF), PEG/PPG-25/25 Dimethicone/Acrylates/t-Butylacrylates Copolymer (Luviflex Silk® von BASF), Acrylic Acid/VP Crosspolymer (Ultrathix P-100® von ISP), Polyvinylcaprolactam (Luviskol Plus® von BASF), PVM/MA Copolymer (Luviform FA® von BASF), PVP (Luviskol® von BASF), PVP/VA Copolymer (Luviskol® von BASF), VP/Dimethylaminoethylmethacrylate Copolymer (Copolymer® von ISP), Sodium Polystyrene Sulfonate (Flexan® von National Starch), VA/Crotonates/Vinyl Neodecanoate Copoylmer (Luviset CAN® von BASF, Resyn® von Natinal Starch), VA/Crotonates/Vinyl Propionate Copolymer (Luviset CAP® von BASF), VA/Butyl Maleate/Isobornyl Acrylate Copolymer (Advantage Plus® von ISP), VA/Crotonates Copolymer (Luviset CA 66® von BASF, Aristoflex® von Clariant), VA/Vinylbutylbenzoate/Crotonates Copolymer (Mexomere PW® von Chimex), Vinylcaprolactam/PVP/Dimethylaminoethyl Methacrylate Copolymer (Advantage® von ISP), VA/Butyl Maleate/Isobornyl Acrylate Copolymer (Advantage Plus® von ISP), Acrylates/C1-2 Succinates/HydroxyAcrylates Copolymer (Allianz LT-120® von ISP), VPNinyl Caprolactam/DMAPA Acrylates Copolymer (Aquaflex SF-40® von ISP), Vinyl Caprolactam/VP/Dimethylaminoethyl Methacrylate Copolymer (Gaffix® von ISP), VP/DMAPA Acrylates Copolymer (Styleze CC-10 von ISP), VP/Acrylates/Lauryl Methacrylate Copolymer (Styleze 2000® von ISP), VP/Methacrylamide/N-Vinyl Imidazole Copolymer (Luviset Clear® von BASF), alle Polyacrylat-Typen (Fixate Plus® und Fixate Superhold von Lubrizol, Luviset Shape® von BASF) und viele mehr. Weitere Polymerklassen, die in Haarfestigern Einsatz finden sind z. B. Polyurethane, z. B. Polyurethane-1 (Luviset PUR® von BASF), Polyurethane-2 (Avalure UR® von Lubrizol), Polyurethane-6 (Luviset SI PUR® von BASF), Polyurethane-14 (and) AMP-Acrylates Copolymer (DynamX von AkzoNobel), Polyester, z.B. Polyester-5 (AQ-Polymere von Eastman), Polyimide, z. B. Polyimid-1 (Aquaflex XL-30® von ISP) und viele mehr.

Ein entscheidender Nachteil der haltgebenden Polymere in Haarfestigern sind die unzureichenden Pflegeeigenschaften: Das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen. Eine Verbesserung in Bezug auf Griff und Kämmbarkeit wird bei Haarfestiger-Formulierungen typischerweise durch die Kombination der genannten Polymere mit kationischen Polymeren, kationischen Tensiden oder Silikonen (beispielsweise cyclische Polydimethylsiloxane (Cyclomethicone), Silikontenside (Polyethermodifizierte Siloxane) vom Typ Dimethicone) erreicht.

Kationische Tenside werden zumeist ausgewählt aus der Gruppe der quaternären Ammoniumverbindungen, insbesondere Benzyltrialkylammoniumsalze oder Alkyltrimethylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Hydroxyethyl Cetyldimonium Phosphate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide.

Kationische Polymere sind unter den Bezeichnungen Polyquaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/- Dimethylaminoethylmethacrylat Copolymer (Polyquaternium-11), Homo- und Copolymere von Dimethyldiallylammoniumchlorid (Polyquaternium-6, -7 oder -22), quaternisierte Cellulosepolymere (Polyquaternium-4, -10), Copolymere aus kationischer Cellulose und Stärke (Polyquaternium-4 / Hydroxypropyl Starch Copolymer), quaternisiertes Vinylpyrrolidon / Dimethylaminopropylmethacrylat / Lauryl Dimethylaminopropylmethacrylat Copolymer (Polyquaternium-55), Vinylpyrrolidon/ Vinylimidazol/Methacrylamid/quaternisiertes Vinylimidazol (Polyquaternium-68), quaternisiertes Vinylcaprolactam/Vinylpyrrolidone/Dimethylaminopropyl methacrylamide /Methacryloylaminopropyllauryldimoniumchloride (Polyquaternium-69) oder quaternisierte Guarderivate.

Einfache linear-polymere Silikone sind nach dem Schema (R₂SiO)ₓ aufgebaut. Systematisch werden die Silikone als *Polyorganosiloxane* bezeichnet. Ein Polymer der allg. Formel 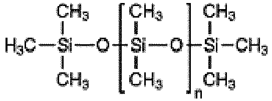 wird als Poly(dimethylsiloxan) bezeichnet, kann aber nach den IUPAC-Regeln zur Benennung linearer organ. Polymerer auch Poly[oxy(dimethylsilylen)] genannt werden; der internat. Freiname der Verbindung ist Dimethicone. Die Zusammensetzung der Siloxan-Einheit ergibt sich unter Berücksichtigung der Tatsache, daß jedes O-Atom als Brückenglied zwischen je zwei Si-Atomen liegt, zu RₙSiO_{(4-n)/2} (n=0, 1, 2 od. 3). Die Anzahl der an ein Si-Zentralatom gebundenen Sauerstoff-Atome bestimmt die Funktionalität der betreffenden Siloxan-Baueinheit. Diese Einheiten sind also monofunktionell, difunktionell, trifunktionell od. tetrafunktionell, wofür die symbol. Schreibweisen M, D, T u. Q eingeführt wurden: [M]=R₃SiO_{1/2}, [D]=R₂SiO_{2/2}, [T]=RSiO_{3/2} u. [Q]=SiO_{4/2}. Vornehmlich wird zwischen vier Verbindungstypen unetrschieden: lineare Polysiloxane vom Bautyp [MDₙM] od. R₃SiO[R₂SiO]ₙSiR₃ (R=CH₃), verzweigte Polysiloxane vom Bautyp [MₙDₘTₗ], cyclische Polysiloxane vom Bautyp [Dₙ] sowie vernetzte Polymere, bei denen mittels T- und Q-Einheiten zwei- bzw. dreidimensionale Netzwerke geknüpft werden. Innerhalb jeder Polymeren-Gruppe läßt sich eine weitere Gliederung je nach der Art der am Silicium-Atom gebundenen Substituenten vornehmen. Das Siloxan-Gerüst kann mit verschiedenartigen Kohlenwasserstoff-Resten beladen sein, es kann außerdem Silicium-funktionelle od. organofunktionelle Gruppen oder beide zugleich enthalten. Die Silikone können je nach Kettenlänge, Verzweigungsgrad und Substituenten niedrigviskos bis hochviskos oder fest sein. Sie sind wärmebeständig, hydrophob und gelten in der Regel als physiologisch verträglich.

Die Länge der Alkylketten bestimmt den Schmelzpunkt und die Härte eines Silikonwachses. Ab C18-Kettenlänge werden die Silikone fest und ihr Schmelzpunkt steigt mit wachsender Kettenlänge an. Als Beispiele können u. a. C24-28 Alkyl Methicone (Abil Wax 9810 P® von Evonik), Cerotyl Dimethicone (Silwax D-226® von Siltech), C26-C28 Alkyl Methicone (Belsil CM 7026VP von Wacker), C30-C45 Alkyl Methicone (Belsil MM8030Flakes® von Wacker), Stearyl Methicone (Wacker Belsil SM 6018® von Wacker), Behenyl Dimethicone (Silwax D-222® von Siltech), Behenoxy Dimethicone (Abil Wax 2440® von Evonik), Cerotyl Dimethicone (Silwax Di-5026® von Siltech) und C26-C38 Alkyl Dimethicone (Wacker Belsil CDM 3526 VP® von Wacker) genannt werden.

In Stylingformulierungen kommen als Pflegestoffe bevorzugt cyclische Polydimethylsiloxane (Cyclomethicone) in Konzentrationen von z. B. 0.1 - 1.0 Gew.-% der Gesamtformulierung oder Silikontenside (Polyethermodifizierte Siloxane) vom Typ Dimethicone Copolyol z. B. in Konz. 0.01 - 1.0 Gew.-% des Gesamtgewichts der Zubereitung zum Einsatz. Cyclomethicone werden u.a. unter der Handelsbezeichnungen Abil K4 von der Gesellschaft Goldschmidt oder z. B. DC 244, DC 245 oder DC 345 von der Gesellschaft Dow Corning angeboten. Dimethicone Copolyole werden z. B. unter der Handelsbezeichnung DC 193 von der Gesellschaft Dow Corning bzw. Belsil DM 6031 von der Gesellschaft Wacker angeboten.

Der Einsatz dieser kationischer Polymere oder kationischer Tenside oder Silikone führt jedoch meistens zu einer deutliche Abnahme der Volumeneigenschaften.

Marktübliche Haarfestiger weisen oftmals eine Reihe von Nachteilen und Unzulänglichkeiten auf. Insbesondere die Produktsensorik (Stabilität, Verteilbarkeit etc.) ist häufig unzureichend. Ursache sind üblicherweise die haltgebenden Polymere. So weisen die Schäume vieler Haarfestiger eine harte Konsistenz auf, sind wässrig und grobblasig, instabil, nicht weich und cremig. Beim Verteilen des Schaumes in den Händen wird dieser oftmals flockig. Ein derartiger Schaum lässt sich schlecht im Haar verteilen, wodurch die eigentliche Leistungsperformance des Produktes zusätzlich verringert wird. Somit geht häufig mit einer Verbesserung der Festigungsleistung eine Verschlechterung der senorischen Eigenschaften einher und andersrum. Zur Verbesserung der Schaumeigenschaften ist der Zusatz von schaumstabilsierenden Additiven, wie z. B. nichtionischen Tensiden üblich.

Als nicht-ionische Tenside werden üblicherweise eingesetzt Alkohole, Alkanolamide, wie Cocamide MEA/ DEA/ MIPA, Aminoxide, wie Cocoamidopropylaminoxid, Ester, die durch Veresterung von Carbonsäuren mit Ethylenoxid, Glycerin, Sorbitan oder anderen Alkoholen entstehen, Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid, Sucroseester, -ether, Polyglycerinester, Diglycerinester, Monoglycerinester, Methylglucosester, sowie Ester von Hydroxysäuren.

Als Treibmittel dienen üblicherweise Kohlenwasserstoffe wie Butan, Isobutan und Propan, Dimethylether oder Fluorkohlenwasserstoffe wie z. B. 1,2-Diflourethan. Andere Treibmittel sind Stickstoff, Kohlendioxid, Distickstoffoxid oder komprimierte Luft.

Bei einer nicht zufriedenstellenden Sensorik schätzen die Verbraucher auch die Leistungen des Produktes geringer ein. Neben der eigentlichen Produktleistung wie Halt oder Volumen beeinflusst die Produktsensorik die Performanceempfindung des Verbrauchers. Deshalb müssen Produktleistung und Sensorik übereinstimmen und gemeinsam gezielt optimiert werden. Es war daher die Aufgabe dieser vorliegenden Erfindung, den Nachteilen des Stands der Technik abzuhelfen und Haarfestiger zu entwickeln, deren sensorische Eigenschaften wie Schaumcharakteristik und die beanspruchte Produktperformance zu optimieren und beides in Einklang zu bringen.

Dabei sollte die Optimierung der Schaumeigenschaften nicht zu einer Verschlechterung der entscheidenden Stylingleistungen wie Volumen und Halt führen, sondern vielmehr die Stylingleistung und dabei insbesondere den Volumeneffekt verbessern. Bislang ging schließlich eine Verbesserung der Schaumsensorik immer mit einer Reduktion der Stylingperformance einher. Darüber hinaus sollte auch das Haargefühl nach Produktauftragung sich geschmeidig und gepflegt anfühlen ohne eine Belegung der Haare oder Rückstände.

Aus der EP 1329212 B1 war bekannt bestimmte Haarbehandlungszubereitungen anzuwenden.

Aus der US 20050222001 A1 waren bestimmte stabile aufgeschäumte Zubereitungen mit Wasser, Gas und bestimmten alkoxylierten Silikonen bekannt.

Aus der WO02102335 war eine Haarzubereitung mit Stylingpolymer, Treibgas und Wachsdispersion bekannt.

Aus der EP 1266650 A1 war ein festes Haarwachsprodukt aus mindestens einem Wachs und/oder mindestens einem wachsartigem Stoff bekannt, das dauerhaft eine Dichte von kleiner oder gleich 0,9 g/ml aufweist.

Aus der DE 10049147 A1 war ein Haarwachsprodukt zur Behandlung oder Erstellung der menschlichen Frisur enthaltend eine wachsförmige Zusammensetzung mit einem Gehalt an mindestens einem Wachs und/oder mindestens einem wachsartigen Stoff, mindestens einem nicht flüchtigen flüssigen hydrophoben Öl und mindestens einem leichtflüchtigen hydrophoben und bei Raumtemperatur flüssigen oder gasförmigen Stoff bekannt

Überraschend und für den Fachmann nicht vorhersehbar hat sich nun herausgestellt, dass eine Frisierzubereitung enthaltend ein festigendes Polymer, ein Silikonwachs und ein Treibgas gewählt unter Butan, Isobutan, Propan, Dimethylether, wobei das festigende Polymer gewählt wird unter PVP/VA, PVP, PVCaprolactam, Carbomere, Acrylates/Hydroxyesters Acrylates Copolymer, Acrylates/Steareth-20 Methacrylate Copolymer, Polyquaternium-4, Polyquaternium-68, Polyquaternium-89, Octylacrylamide/Acrylates/Butylaminoethyl Methacrylat, bevorzugt unter PVP/ VA, Polyquaternium-4, Polyquaternium-68, Octylacrylamide/Acrylates/Butylaminoethyl Methacrylat, besonders bevorzugt PVP/VA, Polyquaternium-4, Polyquaternium-68, und wobei der Silikonwachs a) wenigstens eine endständige Alkylkette mit 18 bis 38 Kohlenstoffatomen, bevorzugt mit 18 bis 26 Kohlenstoffatomen, besonders bevorzugt mit 22 Kohlenstoffatomen und eine Dimethylsiloxankette mit 4 bis 14, bevorzugt 5 bis 10, besonders bevorzugt 6 bis 7 Siliziumatomen aufweist, und b) einen Schmelzpunkt von höchstens 50°C aufweist, den Mängeln des Standes der Technik abhilft. Eine solche Zubereitung ist erhältlich durch des ebenfalls erfindungsgemäße Verfahren zur Herstellung dieser Frisierzubereitung gekennzeichnet durch die Verfahrensschritte a) Zugabe des Silikonwachs und ggf. des Bisbehenoxysiloxan zu den übrigen, bereits gelösten Komponenten der Flüssigphase, dabei wird das Silikonwachs entweder als festes Wachs oder nach vorherigem Aufschmelzen bei 50 - 90°C zugegeben; b) Begasen mit Treibgas, was zum Lösen des Silikonwaches und ggf. des Bisbehenoxysiloxan führt. Das Wachs wird ungelöst und inkompatibel zu der wässrigen Schaumfestigerformulierung gegeben. Überraschenderweise löst es sich nach Zugabe des Treibmittels. Eine derartige Vorgehensweise ist bislang nicht bekannt. Mögliche Treibmittel sind Kohlenwasserstoff-Gemische aus Propan, Butan, iso-Butan (getestete Druckstufen: 2,7 und 3,5 bar), Dimethylether (Druckstufe 4,2 bar). Mögliche Hauptkomponente der flüssigen Produktphase sind Wasser und / oder Ethanol. Der Schmelzpunkt des Wachses muss unter 50°C liegen, sonst lässt es sich nicht durch Zugabe des Treibmittels lösen. Beispiele für solche erfindungsgemäß ungeeigneten Wachse sind C24-28 Alkyl Methicone (Abil Wax 9810 P® von Evonik), Cerotyl Dimethicone (Silwax D-226® von Siltech), C26-C28 Alkyl Methicone (Belsil CM 7026VP von Wacker), C30-C45 Alkyl Methicone (Belsil MM8030Flakes® von Wacker). Erfindungsgemäß sind dagegen die Wachse Stearyl Methicone (Wacker Belsil SM 6018® von Wacker), Behenyl Dimethicone (Silwax D-222® von Siltech), Behenoxy Dimethicone (Abil Wax 2440® von Evonik), Cerotyl Dimethicone (Silwax Di-5026® von Siltech) und C26-C38 Alkyl Dimethicone (Wacker Belsil CDM 3526 VP® von Wacker). Bei all dem soll die flüssige Produktphase keine zu hohe Viskosität aufweisen, die Viskosität liegt erfindungsgemäß unter 15000 mPas bei niedriger Scherrate.

Es ist bevorzugt, wenn ein Silikonwachs in dem Treibgas gelöst ist. Das Silikonwachs weist einen Schmelzpunkt von höchstens 50°C auf. Weiter erfindungsgemäß ist es, wenn die Konzentration an nichtionischen Tensiden 0 bis 5 Gew.-%, bevorzugt 0 bis 3 Gew.-%, besonders bevorzugt 0,1 bis 2 Gew.-% beträgt. Weiter bevorzugt ist es, wenn als nichtionisches Tensid wenigstens eine Verbindung mit einer Fettkette mit 12 bis 18 Kohlenstoffatomen und einer Oligoalkylenoxykette mit 3 bis 20 Alkylenoxyeinheiten enthalten ist. Weiter bevorzugt ist es, wenn der Gehalt an kationischen Tensiden und kationischen Polymeren zusammengenommen 0 bis 8 Gew.-%, bevorzugt 0,1 bis 5 Gew.-% beträgt, bezogen auf den Gehalt an Aktivsubstanz. Das Silikonwachs weist wenigstens eine endständige Alkylkette mit 18 bis 38 Kohlenstoffatomen, bevorzugt mit 18 bis 26 Kohlenstoffatomen, besonders bevorzugt mit 22 Kohlenstoffatomen und eine Dimethylsiloxankette mit 4 bis 14, bevorzugt 5 bis 10, besonders bevorzugt 6 bis 7 Siliziumatomen auf. Weiter bevorzugt ist es, wenn das Silikonwachs Behenoxyoligodimethylsilicon mit 6 bis 7 Siloxaneinheiten ist. Weiter bevorzugt ist es, wenn zusätzlich zum Silikonwachs 2 bis 10 Gew.-% Bisbehenoxysiloxan bezogen auf das Silikonwachs enthalten sind. Weiter bevorzugt ist es, wenn das Silikonwachs in Konzentrationen von 0,1 bis 2 Gew.-% vorliegt. Das festigende Polymer wird unter PVP/VA, PVP, PVCaprolactam, Carbomere, Acrylates/Hydroxyesters Acrylates Copolymer, Acrylates/Steareth-20 Methacrylate Copolymer, Polyquaternium-4, Polyquaternium-68, Polyquaternium-89, Octylacrylamide/Acrylates/Butylaminoethyl Methacrylat, bevorzugt unter PVP/VA, Polyquaternium-4, Polyquaternium-68, Octylacrylamide/Acrylates/Butylaminoethyl Methacrylat, besonders bevorzugt PVP/VA, Polyquaternium-4, Polyquaternium-68 gewählt. Weiter bevorzugt ist es, wenn der Gehalt an festigenden Polymeren zusammengenommen 0,1 bis 15 Gew.-%, bevorzugt 2 bis 10 Gew.-% beträgt, bezogen auf den Gehalt an Aktivsubstanz.

Vorteilhaft können solche Zubereitungen zusätzlich Vitamine, bevorzugt Niacinamide oder Panthenol, den pH-Wert regulierende Rohstoffe, bevorzugt Citronensäure, Aminomethylpropanol, Natronlauge und/oder Triethanolamin, Salze, bevorzugt Natriumchlorid, Kaliumchlorid, Cetrimoniumchlorid oder Hydoxyethyl Cetyldimonium Phosphate un/oder,Silikonöle, bevorzugt Cyclomethicone, Dimethicone und/oder PEG-1 Dimethicone, Kompexbildner, bevorzugt EDTA, kationische Polymere, bevorzugt Polyquaternium-4, Polyquaternium-16 und/oder Polyquaternium-68, Konservierungsmittel, bevorzugt Natriumbenzoat, Phenoxyethanol, Parabene und/oder DMDM Hydantoin, Lösungsvermittler, bevorzugt PEG-40 Hydrogenated Castor Oil und/oder Emulgaoren, bevorzugt Ceteareth-20 enthalten.

### Beispiele

Nachfolgende Beispiele sollen die Erfindung verdeutlichen, ohne sie einzuschränken. Dabei geben die Konzentrationen die Gesamteinsatzmenge an, nicht den Aktivgehalt.

| | **1** | **2** | **3** | **4** | **5** | **6** | **7** |
|---|---|---|---|---|---|---|---|
| Behenoxy Dimethicone¹ | 0,45 | 0,45 | 0,45 | 1 | 0,25 | 0,25 | 0,5 |
| PVP/VA Copolymer² | 5,4 | 5,4 | 5,4 | 12 | 10 | 10 | 3 |
| Polyquaternium-68³ | - | - | - | 5 | 10 | 10 | - |
| Polyquaternium-4⁴ | - | - | - | - | - | - | 1 |
| Cetrimoniumchlorid⁵ | 0,54 | 0,54 | 0,54 | 1,0 | 1,0 | 1,0 | 1,0 |
| Hydroxyethyl Cetyldimonium Phosphate⁶ | - | - | - | - | 0,5 | 0,5 | - |
| Panthenol | 0,1 | 0,1 | 0,1 | 0,1 | 0,1 | 0,1 | 0,1 |
| Niacinamide | 0,02 | 0,02 | 0,02 | 0,01 | 0,02 | 0,02 | 0,02 |
| Citronensäure | 0,02 | 0,02 | 0,02 | 0,2 | 0,2 | 0,2 | 0,3 |
| Natriumbenzoat | 0,18 | - | 0,18 | 0,2 | 0,2 | 0,2 | 0,2 |
| DMDM Hydantoin ¹⁵ | - | 0,18 | - | - | - | - | - |
| Laureth-3⁷ | 0,68 | 0,68 | 0,68 | 0,8 | 1,5 | - | 0,5 |
| Trideceth-12⁸ | 0,68 | 0,68 | 0,68 | 0,8 | - | 1,5 | 0,5 |
| PEG-40 Hydrogenated Castor Oil | 0,32 | 0,32 | 0,32 | 0,2 | 0,2 | 0,2 | 0,2 |
| Ceteareth-20⁹ | - | - | 0,09 | - | - | - | - |
| Parfüm | q.s. | q.s. | q.s. | q.s. | q.s. | q.s. | q.s. |
| Propan/Butan 3,5 bar¹⁰ | 10 | 10 | 10 | 10 | 10 | 10 | 10 |
| Wasser | ad 100 | ad 100 | ad 100 | ad 100 | ad 100 | ad 100 | ad 100 |

| | | | | | | | |
|---|---|---|---|---|---|---|---|
| ¹ Abil Wax 2440, Evonik ² Luviskol® von BASF, 50% aktiv ³ Luviquat Supreme® von BASF, 20% aktiv ⁴ Celquat L-200® von AkzoNobel, 89% aktiv ⁵ Dehyquart A-CA® von Cognis, je 25% aktiv ⁶ Luviquat Mono CP® von BASF, 30% aktiv ⁷ Dehydol LS3 Deo N® von Cognis ⁸ Renex 30® von Croda ⁹ Eumulgin B20® von Cognis ¹⁰ Drivisol 35 A 15 Glydant LTD von Lonza, 54% | | | | | | | |

### Untersuchungen zur Produktleistung

Im Fall von Schaumfestigern stellt neben dem Halt Volumen das wichtigste Kriterium hinsichtlich. Kaufintention und Qualitätsbewertung seitens der Verbraucher dar. Da Kunden einen Volumenbenefit wünschen, finden sich auf dem Markt zahlreiche spezielle Volumen-Stylingprodukte. Nachfolgend finden sich Untersuchungsergebnisse des Volumeneffektes, aber auch der Sensorik der Formel nach Beispiel 2 gegenüber einem Marktprodukt (Nivea Hair Care Volume Lift-Schaumfestiger, von 2005 bis 2008 in Deutschland erhältlich). Nivea Hair Care Volume Lift-Schaumfestiger enthielt kein Silikonwachs. Die Einsatzkonzentration des PVP/VA Copolymers war in diesem Marktprodukt deutlich höher als in der Formel nach Beispiel 2 und enthielt außerdem als Polyquaternium-4 als weiteres Polymer.

Ein signifikanter Anstieg des Volumens konnte *in vitro* mittels Schattenbildmethode gemessen werden, da ein Anstieg der Fläche des Schattenwurfes 60 (Marktprodukt Nivea Hair Care Volume Lift-Schaumfestiger) auf 65 cm² (Beispiel 2) nachgewiesen werden konnte.

Eine Haarstudio-Evaluierung an Probandinnen (n=10) mit feinem bis normalen, ca. kinnlangen Haaren zeigte Vorteile des Beispiel 2 gegenüber (Marktprodukt Nivea Hair Care Volume Lift-Schaumfestiger) hinsichtlich. Gleitvermögen und Haarstruktur im nassen Haar sowie Volumen, Elastizität und Kämmbarkeit im trockenen Haar.

Probandinnen mit feinem bis normalen, ca. kinnlangen Haaren testeten den Prototypen (Beispielrezeptur 2) gegenüber der Marktprodukt Nivea Hair Care Volume Lift-Schaumfestiger (n=51). Signifikante Verbesserungen konnten für folgende Kriterien nachgewiesen werden: cremigerer & pflegenderer Schaum, schöneres Haargefühl, verbesserter Halt, verstärkter Volumeneffekt am Haaransatz, mehr Glanz und somit auch ein insgesamt verbessertes Schaumfestigerprodukt. Interessant ist, dass bei dem Prototypen der Aktivgehalt an Styling-Polymeren reduziert werden konnte (PVP/VA von 4,3 auf 3% aktiv reduziert, PQ-4 von 0,15 auf 0 % aktiv reduziert). Das Wachs führt somit offensichtlich auch zu einem Anstieg des Halts.

| | **8** | **9** | **10** | **11** |
|---|---|---|---|---|
| Behenoxy Dimethicone¹ | 0,45 | 0,5 | 1 | 1 |
| PVP/VA Copolymer ² | 5,4 | 6 | 12 | 12 |
| Polyquaternium-68³ | - | - | 5 | - |
| PVP¹¹ | - | - | - | 3 |
| Cetrimoniumchlorid⁵ | 0,54 | 0,5 | - | - |
| Hydroxyethyl Cetyldimonium Phosphate⁶ | - | - | 1,0 | 1,0 |
| Panthenol | 0,1 | 0,1 | 0,1 | 0,1 |
| Niacinamide | 0,02 | 0,02 | 0,01 | 0,01 |
| Citronensäure | 0,02 | 0,02 | 0,2 | 0,2 |
| Natriumbenzoat | 0,18 | 0,2 | 0,2 | 0,2 |
| Laureth-3⁷ | - | 0,75 | - | 0,8 |
| Laureth-4¹² | 0,5 | - | 0,8 | - |
| Trideceth-12⁸ | 0,8 | 0,75 | 0,8 | 0,8 |
| PEG-40 Hydrogenated Castor Oil | 0,32 | 0,35 | 0,2 | 0,2 |
| Ceteareth-20⁹ | - | 0,1 | - | - |
| Parfüm | q.s. | q.s. | q.s. | q.s. |
| Propan/Butan 3,5 bar¹⁰ | 10 | 8 | 10 | 10 |
| Wasser | ad 100 | ad 100 | ad 100 | ad 100 |

| | | | | |
|---|---|---|---|---|
| ¹¹ Luviskol K 30 Pulver (BASF) ¹² Brij 30® von Croda | | | | |

| | **12** | **13** | **14** | **15** |
|---|---|---|---|---|
| Behenoxy Dimethicone¹ | 1 | 0,5 | 0,5 | 0,45 |
| PVP/VA Copolymer² | 8 | 3 | 3 | 6,3 |
| Polyquaternium-68 ³ | - | - | - | 5 |
| Polyquaternium-16¹³ | - | 7 | 7 | - |
| Polyquaternium-4⁴ | - | - | - | 0,05 |
| Cetrimoniumchlorid⁵ | 2,0 | 1,0 | 1,0 | 0,8 |
| Hydroxyethyl Cetyldimonium Phosphate⁶ | 1,0 | - | - | 0,5 |
| Panthenol | 0,1 | 0,1 | 0,1 | 0,1 |
| Niacinamide | 0,02 | 0,02 | 0,02 | 0,02 |
| Citronensäure | 0,2 | 0,3 | 0,3 | 0,02 |
| Natriumbenzoat | 0,2 | 0,2 | - | 0,18 |
| Laureth-3⁷ | 1,0 | 0,5 | 0,5 | 0,68 |
| Trideceth-12⁸ | 1,0 | 0,5 | 0,5 | 0,68 |
| PEG-40 Hydrogenated Castor Oil | 0,2 | 0,2 | 0,2 | 0,32 |
| Parfüm | q.s. | q.s. | q.s. | q.s. |
| Propan/Butan 3,5 bar ¹⁰ | 10 | 10 | 10 | 10 |
| Wasser | ad 100 | ad 100 | ad 100 | ad 100 |
| Ethanol¹⁴ | - | - | 15 | - |

| | | | | |
|---|---|---|---|---|
| ¹³ Luviquat FC 370 (BASF), 40% aktiv ¹⁴Agraralkohol Surfin Sorte 616 von France Alcools, 96,12% aktiv | | | | |

| | **16** | **17** | **18** | **19** |
|---|---|---|---|---|
| Behenoxy Dimethicone¹ | 1 | 0,25 | 0,5 | 0,5 |
| PVP/VA Copolymer² | 8 | 4 | 3 | 3 |
| AMP-Acrylates/Allyl Methacrylates Copolymer | 6 | - | - | - |
| Acrylates/Hydroxyesters Acrylates Copolymer | - | - | 10 | 10 |
| Acrylates Copolymer | - | 4 | - | - |
| Acrylates/Octylacrylamide Copolymer¹⁵ | - | - | - | 5 |
| Polyquaternium-68 ³ | 3 | 5 | - | - |
| Polyquaternium-16¹³ | 0,5 | - | - | - |
| Aminomethylpropanol | - | 0,1 | 0,1 | 0,1 |
| Cetrimoniumchlorid⁵ | 1,0 | 1,0 | 1,0 | 1,0 |
| Hydroxyethyl Cetyldimonium Phosphate⁶ | - | - | 0,5 | 0,5 |
| Panthenol | 0,1 | 0,1 | 0,1 | 0,1 |
| Niacinamide | - | - | - | - |
| Citronensäure | - | - | - | - |
| Natriumbenzoat | - | - | - | - |
| Phenoxyethanol | 0,5 | 0,5 | 0,5 | 0,5 |
| Parabene | 0,2 | 0,2 | 0,2 | 0,2 |
| Laureth-3⁷ | 1,0 | 1,5 | 0,5 | 0,5 |
| Trideceth-12⁸ | 1,0 | - | 0,5 | 0,5 |
| PEG-40 Hydrogenated Castor Oil | 0,2 | 0,2 | 0,2 | 0,2 |
| Parfüm | q.s. | q.s. | q.s. | q.s. |
| Propan/Butan 3,5 bar ¹⁰ | 10 | 10 | 10 | 10 |
| Wasser | ad 100 | ad 100 | ad 100 | ad 100 |

| | | | | |
|---|---|---|---|---|
| ¹⁵ Amphomer® von National Starch, 97% aktiv | | | | |

## Patentansprüche

1. Frisierzubereitung enthaltend ein festigendes Polymer, ein Silikonwachs und ein Treibgas gewählt unter Butan, Isobutan, Propan, Dimethylether,
wobei das festigende Polymer gewählt wird unter PVP/VA, PVP, PVCaprolactam, Carbomere, Acrylates/Hydroxyesters Acrylates Copolymer, Acrylates/Steareth-20 Methacrylate Copolymer, Polyquaternium-4, Polyquaternium-68, Polyquaternium-89, Octylacrylamide/Acrylates/Butylaminoethyl Methacrylat, bevorzugt unter PVP/VA, Polyquaternium-4, Polyquaternium-68, Octylacrylamide/Acrylates/Butylaminoethyl Methacrylat, besonders bevorzugt PVP/VA, Polyquaternium-4, Polyquaternium-68,
und
wobei der Silikonwachs
a) wenigstens eine endständige Alkylkette mit 18 bis 38 Kohlenstoffatomen, bevorzugt mit 18 bis 26 Kohlenstoffatomen, besonders bevorzugt mit 22 Kohlenstoffatomen und eine Dimethylsiloxankette mit 4 bis 14, bevorzugt 5 bis 10, besonders bevorzugt 6 bis 7 Siliziumatomen aufweist, und
b) einen Schmelzpunkt von höchstens 50°C aufweist.

2. Zubereitung nach Patentanspruch 1, **dadurch gekennzeichnet, dass** ein Silikonwachs in dem Treibgas gelöst ist.

3. Zubereitung nach einem der vorangehenden Patentansprüche, **dadurch gekennzeichnet, dass** die Konzentration an nichtionischen Tensiden 0 bis 5 Gew.-%, bevorzugt 0 bis 3 Gew.-%, besonders bevorzugt 0,1 bis 2 Gew.-% beträgt.

4. Zubereitung nach einem der vorangehenden Patentansprüche, **dadurch gekennzeichnet, dass** als nichtionisches Tensid wenigstens eine Verbindung mit einer Fettkette mit 12 bis 18 Kohlenstoffatomen und einer Oligoalkylenoxykette mit 3 bis 20 Alkylenoxyeinheiten enthalten ist.

5. Zubereitung nach einem der vorangehenden Patentansprüche, **dadurch gekennzeichnet, dass** der Gehalt an kationischen Tensiden und kationischen Polymeren zusammengenommen 0 bis 8 Gew.-%, bevorzugt 0,1 bis 5 Gew.-% beträgt, bezogen auf den Gehalt an Aktivsubstanz.

6. Zubereitung nach einem der vorangehenden Patentansprüche, **dadurch gekennzeichnet, dass** zusätzlich zum Silikonwachs 2 bis 10 Gew.-% Bisbehenoxysiloxan bezogen auf das Silikonwachs enthalten sind.

7. Zubereitung nach einem der vorangehenden,adurch gekennzeichnet, dass das Silikonwachs in Konzentrationen von 0,1 bis 2 Gew.-% vorliegt.

8. Zubereitung nach einem der vorangehenden Patentansprüche, **dadurch gekennzeichnet, dass** der Gehalt an festigenden Polymeren zusammengenommen 0,1 bis 15 Gew.-%, bevorzugt 2 bis 10 Gew.-% beträgt, bezogen auf den Gehalt an Aktivsubstanz.

9. Verfahren zur Herstellung einer Frisierzubereitung nach einem der vorangehenden Patentansprüche **gekennzeichnet durch** die Verfahrensschritte
a) Zugabe des Silikonwachs und ggf. des Bisbehenoxysiloxan zu den übrigen, bereits gelösten Komponenten der Flüssigphase; dabei wird das Silikonwachs entweder als festes Wachs oder nach vorherigem Aufschmelzen bei 50 - 90°C zugegeben;
b) Begasen mit Treibgas, was zum Lösen des Silikonwaches und ggf. des Bisbehenoxysiloxans führt.

## Claims

1. Styling preparation comprising a fixing polymer, a silicone wax and a propellant selected from butane, isobutane, propane, dimethyl ether,
wherein the fixing polymer is selected from PVP/VA, PVP, PVCaprolactam, carbomers, acrylates/hydroxyesters acrylates copolymer, acrylates/steareth-20 methacrylate copolymer, polyquaternium-4, polyquaternium-68, polyquaternium-89, octylacrylamide/acrylates/butylaminoethyl methacrylate, preferably from PVP/VA, polyquaternium-4, polyquaternium-68, octylacrylamide/acrylates/butylaminoethyl methacrylate, particularly preferably PVP/VA, polyquaternium-4, polyquaternium-68,
and
wherein the silicone wax
a) has at least one terminal alkyl chain having 18 to 38 carbon atoms, preferably having 18 to 26 carbon atoms, particularly preferably having 22 carbon atoms and a dimethylsiloxane chain having 4 to 14, preferably 5 to 10, particularly preferably 6 to 7 silicon atoms, and
b) has a melting point of at most 50°C.

2. Preparation according to patent Claim 1, **characterized in that** a silicone wax is dissolved in the propellant.

3. Preparation according to either of the preceding patent claims, **characterized in that** the concentration of non-ionic surfactants is 0 to 5% by weight, preferably 0 to 3% by weight, particularly preferably 0.1 to 2% by weight.

4. Preparation according to any of the preceding patent claims, **characterized in that** at least one compound with a fatty chain having 12 to 18 carbon atoms and an oligoalkyleneoxy chain having 3 to 20 alkyleneoxy units is present as non-ionic surfactant.

5. Preparation according to any of the preceding patent claims, **characterized in that** the combined content of cationic surfactants and cationic polymers is 0 to 8% by weight, preferably 0.1 to 5% by weight, based on the active substance content.

6. Preparation according to any of the preceding patent claims, **characterized in that**, in addition to the silicone wax, 2 to 10% by weight bisbehenoxysiloxane is present, based on the silicone wax.

7. Preparation according to any of the preceding claims, **characterized in that** the silicone wax is present at concentrations of 0.1 to 2% by weight.

8. Preparation according to any of the preceding patent claims, **characterized in that** the combined content of fixing polymers is 0.1 to 15% by weight, preferably 2 to 10% by weight, based on the active substance content.

9. Method for preparing a styling preparation according to any of preceding patent claims, **characterized by** the method steps of
a) adding the silicone wax and optionally the bisbehenoxysiloxane to the other components already dissolved in the liquid phase; here the silicone wax is added either as a solid or after prior melting at 50 - 90°C;
b) gassing with propellant leading to dissolution of the silicone wax and optionally the bisbehenoxysiloxane.

## Revendications

1. Préparation pour la coiffure, contenant un polymère fixant, une cire de silicone et un gaz gonflant choisi parmi le butane, l'isobutane, le propane, l'éther diméthylique,
le polymère fixant étant choisi parmi la PVP/VA, la PVP, le PV-caprolactame, les carbomères, le copolymère d'acrylate/acrylate d'hydroxyester, le copolymère d'acrylate/méthacrylate de stéareth-20, le polyquaternium-4, le polyquaternium-68, le polyquaternium-89, l'octylacrylamide/acrylate/méthacrylate de butylaminoéthyle, de préférence parmi la PVP/VA, le polyquaternium-4, le polyquaternium-68, l'octylacrylamide/acrylate/méthacrylate de butylaminoéthyle, de manière particulièrement préférée la PVP/VA, le polyquaternium-4, le polyquaternium-68, et la cire de silicone
a) comprenant au moins une chaîne alkyle terminale de 18 à 38 atomes de carbone, de préférence de 18 à 16 atomes de carbone, de manière particulièrement préférée de 22 atomes de carbone, et une chaîne diméthylsiloxane de 4 à 14, de préférence 5 à 10, de manière particulièrement préférée 6 à 7 atomes de silicium, et
b) présentant un point de fusion d'au plus 50 °C.

2. Préparation selon la revendication 1, **caractérisée en ce qu'**une cire de silicone est dissoute dans le gaz gonflant.

3. Préparation selon l'une quelconque des revendications précédentes, **caractérisée en ce que** la concentration en tensioactifs non ioniques est de 0 à 5 % en poids, de préférence de 0 à 3 % en poids, de manière particulièrement préférée de 0,1 à 2 % en poids.

4. Préparation selon l'une quelconque des revendications précédentes, **caractérisée en ce qu'**au moins un composé comprenant une chaîne grasse de 12 à 18 atomes de carbone et une chaîne oligoalkylène-oxy de 3 à 20 unités alkylène-oxy est contenu en tant que tensioactif non ionique.

5. Préparation selon l'une quelconque des revendications précédentes, **caractérisée en ce que** la teneur en tensioactifs cationiques et en polymères cationiques pris ensemble est de 0 à 8 % en poids, de préférence de 0,1 à 5 % en poids, par rapport à la teneur en substance active.

6. Préparation selon l'une quelconque des revendications précédentes, **caractérisée en ce qu'**en plus de la cire de silicone, 2 à 10 % en poids de bisbéhénoxysiloxane est contenu, par rapport à la cire de silicone.

7. Préparation selon l'une quelconque des revendications précédentes, **caractérisée en ce que** la cire de silicone est présente en concentrations de 0,1 à 2 % en poids.

8. Préparation selon l'une quelconque des revendications précédentes, **caractérisée en ce que** la teneur en polymères fixant pris ensemble est de 0,1 à 15 % en poids, de préférence de 2 à 10 % en poids, par rapport à la teneur en substance active.

9. Procédé de fabrication d'une préparation pour la coiffure selon l'une quelconque des revendications précédentes, **caractérisé par** les étapes de procédé suivantes :
a) l'ajout de la cire de silicone et éventuellement du bisbéhénoxysiloxane aux autres composants déjà dissous de la phase liquide ; la cire de silicone étant ajoutée sous la forme d'une cire solide ou après fusion préalable à 50 à 90 °C ;
b) le gazage avec un gaz gonflant, qui conduit à la dissolution de la cire de silicone et éventuellement du bisbéhénoxysiloxane.
